# EUROPEAN PATENT SPECIFICATION

(11) **EP 3 226 842 B1**
(45) Date of publication and mention of the grant of the patent: **25.11.2020**
(21) Application number: 15817642.0
(22) Date of filing: 03.12.2015
(51) Int. Cl.: A61K 9/14, A61K 9/20, A61P 13/08, A61P 35/00, A61P 35/04

(54) **ANTICANCER COMPOSITIONS**
ANTIKREBSZUSAMMENSETZUNGEN
COMPOSITIONS ANTICANCÉREUSES

(30) Priority: 05.12.2014 EP 14196591
(43) Date of publication of application: 11.10.2017
(73) Proprietor: Aragon Pharmaceuticals, Inc., San Diego, CA 92130 (US)
(72) Inventor: VERRECK, Geert, 2340 Beerse (BE)
(74) Representative: Carpmaels & Ransford LLP
(86) International application number: PCT/US2015/063667
(87) International publication number: WO 2016/090101

(56) References cited:
- WO-A1-2013/152342
- WO-A1-2015/023710
- WO-A1-2015/118015

## Description

The present invention concerns pharmaceutical formulations of ARN-509, which can be administered to a mammal, in particular a human, suffering from an androgen receptor (AR)-related disease or condition, in particular cancer, more in particular prostate cancer, including but not limited to castration-resistant prostate cancer, metastatic castration resistant prostate cancer, chemotherapy-naive metastatic castration resistant prostate cancer, biochemically relapsed hormone sensitive prostate cancer, or high-risk, non-metastatic castration-resistant prostate cancer. These formulations comprise a solid dispersion of ARN-509 and a poly(meth)acrylate copolymer. In one aspect, the solid dispersion of ARN-509 and a poly(meth)acrylate copolymer is obtainable, in particular is obtained, by melt-extruding a mixture comprising ARN-509 and a poly(meth)acrylate copolymer and optionally subsequently milling said melt-extruded mixture. In one aspect, the solid dispersion of ARN-509 and a poly(meth)acrylate copolymer is obtainable, in particular is obtained, by spray drying a mixture comprising ARN-509 and a poly(meth)acrylate copolymer in a suitable solvent.

The solid dispersion of ARN-509 and a poly(meth)acrylate copolymer may be further formulated with a pharmaceutically acceptable carrier into a pharmaceutical formulation, such formulation providing improved stability or improved shelf life. The formulation of the present invention provides for a fast drug release. With the formulation of the present invention the pill burden for the patient, in particular the cancer patient, can be reduced, and hence therapy adherence and therapy efficiency can be improved.

### FIGURES

- Fig. 1 :: XRD pattern of ARN-509 Form B.
- Fig. 2 :: IR spectrum of ARN-509 Form B.
- Fig. 3 :: DSC curve of ARN-509 Form B.

### DETAILED DESCRIPTION

ARN-509 is a potent and specific antagonist of the androgen receptor (AR). ARN-509's mechanism of action is antagonism of androgen receptor signaling through inhibition of AR nuclear translocation and DNA binding to androgen response elements.

The actions of androgens with androgen receptors have been implicated in a number of diseases or conditions, such as androgen dependent cancers, virilization in women, and acne, among others. Compounds that diminish the effects of androgens with androgen receptors and/or lower the concentrations of androgen receptors find use in the treatment of diseases or conditions in which androgen receptors play a role.

AR-related diseases or conditions include, but are not limited to, benign prostate hyperplasia, hirsutism, acne, adenomas and neoplasias of the prostate, benign or malignant tumor cells containing the androgen receptor, hyperpilosity, seborrhea, endometriosis, polycystic ovary syndrome, androgenic alopecia, hypogonadism, osteoporosis, suppression of spermatogenesis, libido, cachexia, anorexia, androgen supplementation for age related decreased testosterone levels, prostate cancer, breast cancer, endometrial cancer, uterine cancer, hot flashes, Kennedy's disease muscle atrophy and weakness, skin atrophy, bone loss, anemia, arteriosclerosis, cardiovascular disease, loss of energy, loss of well-being, type 2 diabetes, and abdominal fat accumulation. Given the central role of AR in prostate cancer development and progression, ARN-509 is useful for the treatment of cancer, in particular prostate cancer, including but not limited to castration-resistant prostate cancer, metastatic castration resistant prostate cancer, chemotherapy-naive metastatic castration resistant prostate cancer, biochemically relapsed hormone sensitive prostate cancer, or high-risk, non-metastatic castration-resistant prostate cancer.

The chemical structure of ARN-509 is:

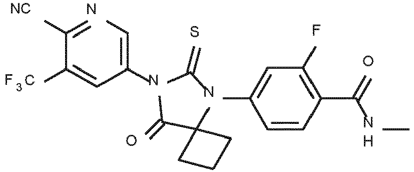

ARN-509 or 4-[7-(6-cyano-5-trifluoromethylpyridin-3-yl)-8-oxo-6-thioxo-5,7-diazaspiro[3.4]oct-5-yl]-2-fluoro-*N*-methylbenzamide is currently in clinical development as a nonaqueous, lipid-based solution that is filled into softgel capsules, each containing 30 mg ARN-509. The daily dose being studied is 240 mg/day by oral administration (or 8 softgel capsules). It has been found that in use, the softgel capsules containing ARN-509 have a shelf life of only 6 months and need cold chain storage.

WO2013/152342 discloses methods and compositions for treating cancer comprising administering to a patient a combination regimen comprising a compound disclosed therein and an androgen receptor antagonist.

An aspect of the invention are pharmaceutical formulations, in particular solid pharmaceutical formulations, more in particular solid pharmaceutical formulations for oral adminstration of ARN-509, where such formulations have an improved stability, a longer shelf life, provide for a fast drug release or provide for a reduced pill burden for the patient, in particular the cancer patient. The pharmaceutical formulations of the present invention provide a means to increase therapy adherence and therapy efficiency.

The invention provides a solid dispersion comprising ARN-509 and a poly(meth)acrylate copolymer.

Copolymers derived from esters of acrylic and methacrylic acid (poly(meth)acrylates) are known in the industry as Eudragit®. Eudragit® is the brand name for a diverse range of poly(meth)acrylate-based copolymers. Different grades are available. In an aspect of the invention, the Eudragit® in the dispersions with ARN-509 is Eudragit® L 100-55 which contains an anionic copolymer based on methacrylic acid and ethyl acrylate (CAS number 25212-88-8; Chemical/IUPAC name: Poly(methacrylic acid-co-ethyl acrylate) 1:1) (Evonik Industries). In an aspect of the invention, the Eudragit® in the dispersions with ARN-509 is Eudragit® E 100 which is a cationic copolymer based on dimethylaminoethyl methacrylate, butyl methacrylate, and methyl methacrylate (CAS number 24938-16-7; Chemical/ IUPAC name: Poly(butyl methacrylate-co-(2-dimethylaminoethyl) methacrylate-co-methyl methacrylate) 1:2:1 (Evonik Industries).

An aspect of the invention is a solid dispersion comprising ARN-509 and Eudragit® L 100-55.
An aspect of the invention is a solid dispersion comprising ARN-509 and Eudragit® E 100.

An aspect of the invention is a solid dispersion consisting of ARN-509 and a poly(meth)acrylate copolymer.

An aspect of the invention is a solid dispersion consisting of ARN-509 and Eudragit® L 100-55.

An aspect of the invention is a solid dispersion consisting of ARN-509 and Eudragit® E 100.

A preferred grade of the poly(meth)acrylate copolymer in the solid dispersions of the invention is Eudragit® L 100-55.

In an aspect of the invention, the weight-by-weight ratio of ARN-509 : poly(meth)acrylate copolymer in the solid dispersion as described herein is in the range from 1:1 to 1:10, preferably from 1:1 to 1:5, more preferably from 1:1 to 1:3 or from 1:2 to 1:3. In an aspect of the invention, the weight-by-weight ratio of ARN-509 : poly(meth)acrylate copolymer is 1:2. In an aspect of the invention, the weight-by-weight ratio of ARN-509 : poly(meth)acrylate copolymer is 1:3. In an aspect of the invention, the weight-by-weight ratio of ARN-509 : Eudragit® L 100-55 is 1:2. In an aspect of the invention, the weight-by-weight ratio of ARN-509 : Eudragit® L 100-55 is 1:3. In an aspect of the invention, the weight-by-weight ratio of ARN-509 : Eudragit® E 100 is 1:2. In an aspect of the invention, the weight-by-weight ratio of ARN-509 : Eudragit® E 100 is 1:3.

An aspect of the invention is a particle consisting of a solid dispersion as described herein.

An aspect of the invention is a particle consisting of a solid dispersion comprising ARN-509 and a poly(meth)acrylate copolymer, in particular wherein the weight-by-weight ratio of ARN-509 : poly(meth)acrylate copolymer is 1:2 or 1:3.

An aspect of the invention is a particle consisting of a solid dispersion comprising ARN-509 and Eudragit® L 100-55, in particular wherein the weight-by-weight ratio of ARN-509 : Eudragit® L 100-55 is 1:2 or 1:3.

An aspect of the invention is a particle consisting of a solid dispersion comprising ARN-509 and Eudragit® E 100, in particular wherein the weight-by-weight ratio of ARN-509 : Eudragit® E 100 is 1:2 or 1:3.

An aspect of the invention is a particle consisting of a solid dispersion consisting of ARN-509 and a poly(meth)acrylate copolymer, in particular wherein the weight-by-weight ratio of ARN-509 : poly(meth)acrylate copolymer is 1:2 or 1:3.

An aspect of the invention is a particle consisting of a solid dispersion consisting of ARN-509 and Eudragit® L 100-55, in particular wherein the weight-by-weight ratio of ARN-509 : Eudragit® L 100-55 is 1:2 or 1:3.

An aspect of the invention is a particle consisting of a solid dispersion consisting of ARN-509 and Eudragit® E 100, in particular wherein the weight-by-weight ratio of ARN-509 : Eudragit® E 100 is 1:2 or 1:3.

An aspect of the invention is a particle comprising a solid dispersion as described hereinabove.

An aspect of the invention is a particle comprising a solid dispersion comprising ARN-509 and a poly(meth)acrylate copolymer, in particular wherein the weight-by-weight ratio of ARN-509 : poly(meth)acrylate copolymer is 1:2 or 1:3.

An aspect of the invention is a particle comprising a solid dispersion comprising ARN-509 and Eudragit® L 100-55, in particular wherein the weight-by-weight ratio of ARN-509 : Eudragit® L 100-55 is 1:2 or 1:3.

An aspect of the invention is a particle comprising a solid dispersion comprising ARN-509 and Eudragit® E 100, in particular wherein the weight-by-weight ratio of ARN-509 : Eudragit® E 100 is 1:2 or 1:3.

An aspect of the invention is a particle comprising a solid dispersion consisting of ARN-509 and a poly(meth)acrylate copolymer, in particular wherein the weight-by-weight ratio of ARN-509 : poly(meth)acrylate copolymer is 1:2 or 1:3.

An aspect of the invention is a particle comprising a solid dispersion consisting of ARN-509 and Eudragit® L 100-55, in particular wherein the weight-by-weight ratio of ARN-509 : Eudragit® L 100-55 is 1:2 or 1:3.

An aspect of the invention is a particle comprising a solid dispersion consisting of ARN-509 and Eudragit® E 100, in particular wherein the weight-by-weight ratio of ARN-509 : Eudragit® E 100 is 1:2 or 1:3.

In an aspect of the invention, the particles as described herein are obtainable, in particular are obtained, by melt-extruding a mixture comprising ARN-509 and a poly(meth)acrylate copolymer and subsequently milling said melt-extruded mixture. In an aspect, the particles as described herein are obtainable, in particular are obtained, by melt-extruding a mixture consisting of ARN-509 and a poly(meth)acrylate copolymer and subsequently milling said melt-extruded mixture. In an aspect, the weight-by-weight ratio of ARN-509 : poly(meth)acrylate copolymer is 1:2 or 1:3.

In an aspect of the invention, the particles as described herein are obtainable, in particular are obtained, by melt-extruding a mixture comprising ARN-509 and Eudragit® L 100-55 and subsequently milling said melt-extruded mixture. In an aspect, the particles as described herein are obtainable, in particular are obtained, by melt-extruding a mixture consisting of ARN-509 and Eudragit® L 100-55 and subsequently milling said melt-extruded mixture. In an aspect, the weight-by-weight ratio of ARN-509 : Eudragit® L 100-55 is 1:2 or 1:3.

In an aspect of the invention, the particles as described herein are obtainable, in particular are obtained, by melt-extruding a mixture comprising ARN-509 and Eudragit® E 100 and subsequently milling said melt-extruded mixture. In an aspect, the particles as described herein are obtainable, in particular are obtained, by melt-extruding a mixture consisting of ARN-509 and Eudragit® E 100 and subsequently milling said melt-extruded mixture. In an aspect, the weight-by-weight ratio of ARN-509 : Eudragit® E 100 is 1:2 or 1:3.

In an aspect of the invention, the particles as described herein are obtainable, in particular are obtained, by spray drying a mixture comprising ARN-509 and a poly(meth)acrylate copolymer in a suitable solvent. In an aspect, the particles as described herein are obtainable, in particular are obtained, by spray drying a mixture consisting of ARN-509 and a poly(meth)acrylate copolymer in a suitable solvent. In an aspect, the weight-by-weight ratio of ARN-509 : poly(meth)acrylate copolymer is 1:2 or 1:3.

In an aspect of the invention, the particles as described herein are obtainable, in particular are obtained, by spray drying a mixture comprising ARN-509 and Eudragit® L 100-55 in a suitable solvent. In an aspect, the particles as described herein are obtainable, in particular are obtained, by spray drying a mixture consisting of ARN-509 and Eudragit® L 100-55 in a suitable solvent. In an aspect, the weight-by-weight ratio of ARN-509 : Eudragit® L 100-55 is 1:2 or 1:3.

In an aspect of the invention, the particles as described herein are obtainable, in particular are obtained, by spray drying a mixture comprising ARN-509 and Eudragit® E 100 in a suitable solvent. In an aspect, the particles as described herein are obtainable, in particular are obtained, by spray drying a mixture consisting of ARN-509 and Eudragit® E 100 in a suitable solvent. In an aspect, the weight-by-weight ratio of ARN-509 : Eudragit® E 100 is 1:2 or 1:3.

An aspect of the invention is a pharmaceutical formulation comprising a pharmaceutically acceptable carrier and a solid dispersion as described herein.

An aspect of the invention is a pharmaceutical formulation comprising a pharmaceutically acceptable carrier and particles as described herein.

An aspect of the invention is a pharmaceutical formulation comprising a pharmaceutically acceptable carrier and a solid dispersion, said solid dispersion comprising ARN-509 and a poly(meth)acrylate copolymer. An aspect of the invention is a pharmaceutical formulation comprising a pharmaceutically acceptable carrier and a solid dispersion, said solid dispersion consisting of ARN-509 and a poly(meth)acrylate copolymer. In an aspect, the weight-by-weight ratio of ARN-509 : poly(meth)acrylate copolymer is 1:2 or 1:3.

An aspect of the invention is a pharmaceutical formulation comprising a pharmaceutically acceptable carrier and a solid dispersion, said solid dispersion comprising ARN-509 and Eudragit® L 100-55. An aspect of the invention is a pharmaceutical formulation comprising a pharmaceutically acceptable carrier and a solid dispersion, said solid dispersion consisting of ARN-509 and Eudragit® L 100-55.

In an aspect, the weight-by-weight ratio of ARN-509 : Eudragit® L 100-55 is 1:2 or 1:3.

An aspect of the invention is a pharmaceutical formulation comprising a pharmaceutically acceptable carrier and a solid dispersion, said solid dispersion comprising ARN-509 and Eudragit® E 100. An aspect of the invention is a pharmaceutical formulation comprising a pharmaceutically acceptable carrier and a solid dispersion, said solid dispersion consisting of ARN-509 and Eudragit® E 100. In an aspect, the weight-by-weight ratio of ARN-509 : Eudragit® E 100 is 1:2 or 1:3.

An aspect of the invention is a pharmaceutical formulation comprising a pharmaceutically acceptable carrier and particles comprising a solid dispersion, said solid dispersion comprising ARN-509 and a poly(meth)acrylate copolymer. An aspect of the invention is a pharmaceutical formulation comprising a pharmaceutically acceptable carrier and particles comprising a solid dispersion, said solid dispersion consisting of ARN-509 and a poly(meth)acrylate copolymer. In an aspect, the weight-by-weight ratio of ARN-509 : poly(meth)acrylate copolymer is 1:2 or 1:3. In an aspect, the particles are obtainable, in particular are obtained, by spray drying as described herein. In an aspect, the particles are obtainable, in particular are obtained, by melt extrusion as described herein.

An aspect of the invention is a pharmaceutical formulation comprising a pharmaceutically acceptable carrier and particles consisting of a solid dispersion, said solid dispersion comprising ARN-509 and a poly(meth)acrylate copolymer. An aspect of the invention is a pharmaceutical formulation comprising a pharmaceutically acceptable carrier and particles consisting of a solid dispersion, said solid dispersion consisting of ARN-509 and a poly(meth)acrylate copolymer. In an aspect, the particles are obtainable, in particular are obtained, by spray drying as described herein. In an aspect, the particles are obtainable, in particular are obtained, by melt extrusion as described herein.

An aspect of the invention is a pharmaceutical formulation comprising a pharmaceutically acceptable carrier and particles comprising a solid dispersion, said solid dispersion comprising ARN-509 and Eudragit® L 100-55. An aspect of the invention is a pharmaceutical formulation comprising a pharmaceutically acceptable carrier and particles comprising a solid dispersion, said solid dispersion consisting of ARN-509 and Eudragit® L 100-55. In an aspect, the weight-by-weight ratio of ARN-509 : Eudragit® L 100-55 is 1:2 or 1:3. In an aspect, the particles are obtainable, in particular are obtained, by spray drying as described herein. In an aspect, the particles are obtainable, in particular are obtained, by melt extrusion as described herein.

An aspect of the invention is a pharmaceutical formulation comprising a pharmaceutically acceptable carrier and particles comprising a solid dispersion, said solid dispersion comprising ARN-509 and Eudragit® E 100. An aspect of the invention is a pharmaceutical formulation comprising a pharmaceutically acceptable carrier and particles comprising a solid dispersion, said solid dispersion consisting of ARN-509 and Eudragit® E 100. In an aspect, the weight-by-weight ratio of ARN-509 : Eudragit® E 100 is 1:2 or 1:3. In an aspect, the particles are obtainable, in particular are obtained, by spray drying as described herein. In an aspect, the particles are obtainable, in particular are obtained, by melt extrusion as described herein.

An aspect of the invention is a pharmaceutical formulation comprising a pharmaceutically acceptable carrier and particles consisting of a solid dispersion, said solid dispersion comprising ARN-509 and Eudragit® L 100-55. An aspect of the invention is a pharmaceutical formulation comprising a pharmaceutically acceptable carrier and particles consisting of a solid dispersion, said solid dispersion consisting of ARN-509 and Eudragit® L 100-55. In an aspect, the weight-by-weight ratio of ARN-509 : Eudragit® L 100-55 is 1:2 or 1:3. In an aspect, the particles are obtainable, in particular are obtained, by spray drying as described herein. In an aspect, the particles are obtainable, in particular are obtained, by melt extrusion as described herein.

An aspect of the invention is a pharmaceutical formulation comprising a pharmaceutically acceptable carrier and particles consisting of a solid dispersion, said solid dispersion comprising ARN-509 and Eudragit® E 100. An aspect of the invention is a pharmaceutical formulation comprising a pharmaceutically acceptable carrier and particles consisting of a solid dispersion, said solid dispersion consisting of ARN-509 and Eudragit® E 100. In an aspect, the weight-by-weight ratio of ARN-509 : Eudragit® E 100 is 1:2 or 1:3. In an aspect, the particles are obtainable, in particular are obtained, by spray drying as described herein. In an aspect, the particles are obtainable, in particular are obtained, by melt extrusion as described herein.

An aspect of the invention is a solid dispersion as described herein wherein no surfactant is present.

An aspect of the invention is a particle as described herein wherein no surfactant is present.

An aspect of the invention is a pharmaceutical formulation as described herein wherein no surfactant is present.

An aspect of the invention is a solid dispersion as described herein wherein ARN-509 is the only active pharmaceutical ingredient.

An aspect of the invention is a particle as described herein wherein ARN-509 is the only active pharmaceutical ingredient.

An aspect of the invention is a pharmaceutical formulation as described herein wherein ARN-509 is the only active pharmaceutical ingredient.

In the solid dispersions or particles or pharmaceutical formulations as described herein ARN-509 is present in base form or as a pharmaceutically acceptable addition salt, such as a pharmaceutically acceptable acid addition salt. Preferably, ARN-509 is present in base form.

The pharmaceutically acceptable addition salts are meant to comprise the therapeutically active non-toxic salt forms. The acid addition salt forms can be obtained by treating the base form of ARN-509 with an appropriate acid, such as inorganic acids, including but not limited to, hydrohalic acids, e.g. hydrochloric acid, hydrobromic acid and the like acids; sulfuric acid; nitric acid; phosphoric acid; metaphosphoric acid and the like acids; or organic acids, including but not limited to, acetic acid, trifluoroacetic acid, trimethylacetic acid, propanoic acid, hydroxyacetic acid, 2-hydroxypropanoic acid, 2-oxopropanoic acid, glycolic acid, oxalic acid, malonic acid, succinic acid, maleic acid, fumaric acid, malic acid, mandelic acid, tartaric acid, 2-hydroxy-1,2,3-propanetricarboxylic acid, methanesulfonic acid, ethanesulfonic acid, 1,2-ethanedisulfonic acid, 2- hydroxyethanesulfonic acid, benzoic acid, cinnamic acid, hydrocinnamic acid, benzenesulfonic acid, 4-methylbenzenesulfonic acid, 2- naphthalenesulfonic acid, cyclohexanesulfamic acid, 2-hydroxybenzoic acid, 4-amino-2-hydroxybenzoic acid, hexanoic acid, cyclopentanepropionic acid, 3-(4-hydroxybenzoyl)benzoic acid, 4-methylbicyclo-[2.2.2]oct-2-ene-1-carboxylic acid, glucoheptonic acid, tertiary butylacetic acid, lauryl sulfuric acid, gluconic acid, glutamic acid, hydroxynaphthoic acid, stearic acid, muconic acid, butyric acid, phenylacetic acid, phenylbutyric acid, valproic acid, and the like acids.

Conversely said salt forms can be converted by treatment with an appropriate base into the free base form.

Also included are the hydrates, the solvent addition forms and mixtures thereof which ARN-509 or its salts are able to form. Examples of such forms are e.g. hydrates, alcoholates and the like, for instance an ethanolate.

In general, doses employed for adult human treatment are typically in the range from 0.01 mg to 5000 mg per day. In one aspect, doses employed for adult human treatment are from about 1 mg to about 1000 mg per day. In another aspect, doses employed for adult human treatment are from about 100 mg to about 500 mg per day. In another aspect, the dose employed for adult human treatment is 240 mg per day. The exact dosage and frequency of administration of ARN-509 may depend on the particular condition being treated, the severity of the condition being treated, the age, weight and general physical condition of the particular patient as well as other medication the individual may be taking, as is known to those skilled in the art. Furthermore, it is evident that said daily amounts may be lowered or increased depending on the response of the treated subject and/or depending on the evaluation of the physician prescribing ARN-509. The doses mentioned herein are therefore only a guideline and are not intended to limit the scope or use of the invention to any extent. In an aspect of the invention, the daily dose is conveniently presented in a single dose or in divided doses administered simultaneously (or over a short period of time) or at appropriate intervals, for example as two, three, four or more sub-doses per day. In an aspect of the invention, the daily dose is administered in 4 divided doses. In an aspect of the invention, the daily dose is administered in 4 divided doses administered simultaneously (or over a short period of time). In an aspect of the invention, the daily dose is administered in 3 divided doses. In an aspect of the invention, the daily dose is administered in 3 divided doses administered simultaneously (or over a short period of time). In an aspect of the invention, the daily dose is administered in 2 divided doses. In an aspect of the invention, the daily dose is administered in 2 divided doses administered simultaneously (or over a short period of time).

In an aspect of the invention, the pharmaceutical formulation comprises 240 mg of ARN-509.
In an aspect of the invention, the pharmaceutical formulation comprises 120 mg of ARN-509.
In an aspect of the invention, the pharmaceutical formulation comprises 60 mg of ARN-509.

In an aspect of the invention, the pharmaceutical formulation comprises 240 mg of ARN-509. The pharmaceutical formulation is administered once daily.

In an aspect of the invention, the pharmaceutical formulation comprises 120 mg of ARN-509. Two of said formulations are administered daily, preferably simultaneously (or over a short period of time).

In an aspect of the invention, the pharmaceutical formulation comprises 60 mg of ARN-509. Four of said formulations are administered daily, preferably simultaneously (or over a short period of time).

The formulation of the present invention can also be used in combination with another anticancer agent, in particular with another anti prostate cancer agent, more in particular with an androgen biosynthesis inhibitor, that inhibits 17 α-hydroxylase/C17,20-lyase (CYP17), in particular abiraterone acetate. The formulation of the present invention can further be combined with prednisone.
Thus, the present invention also relates to a combination of a pharmaceutical formulation according to the invention and another anticancer agent, in particular another anti prostate cancer agent, more in particular an androgen biosynthesis inhibitor, that inhibits 17 α-hydroxylase/C17,20-lyase (CYP17), in particular abiraterone acetate.
Said combination may further comprise prednisone.

The term "a solid dispersion" means a system in a solid state (as opposed to a liquid or gaseous state) comprising at least two components, wherein one component is dispersed more or less evenly throughout the other component or components. When said dispersion of the components is such that the system is chemically and physically uniform or homogenous throughout or consists of one phase as defined in thermodynamics, such a solid dispersion will be called "a solid solution" herein. Solid solutions are preferred physical systems because the components therein are usually readily bioavailable to the organisms to which they are administered. This advantage can probably be explained by the ease with which said solid solutions can form liquid solutions when contacted with a liquid medium such as gastric juice. The ease of dissolution may be attributed at least in part to the fact that the energy required for dissolution of the components from a solid solution is less than that required for the dissolution of components from a crystalline or microcrystalline solid phase.

The term "a solid dispersion" also comprises dispersions which are less homogenous throughout than solid solutions. Such dispersions are not chemically and physically uniform throughout or comprise more than one phase. For example, the term "a solid dispersion" also relates to a system in a solid state comprising at least two components (a) and (b) and having domains or small regions wherein amorphous, microcrystalline or crystalline (a), or amorphous, microcrystalline or crystalline (b), or both, are dispersed more or less evenly in another phase comprising (b), or (a), or a solid solution comprising (a) and (b). Said domains are regions distinctively marked by some physical feature, small in size compared to the size of the system as a whole, and evenly and randomly distributed throughout the system.

Preferred are solid dispersions or particles as described herein wherein ARN-509 is in a non-crystalline phase as these have an intrinsically faster dissolution rate than those wherein part or all of ARN-509 is in a microcrystalline or crystalline form.

Alternatively, the solid dispersions may be in the form of a dispersion wherein amorphous or microcrystalline ARN-509 or amorphous or microcrystalline poly(meth)acrylate copolymer is dispersed more or less evenly in a solid solution comprising ARN-509 and a poly(meth)acrylate copolymer.

In an aspect of the invention ARN-509 is present in the solid dispersions as described herein in amorphous form.

In an aspect of the invention the solid dispersion as described herein is a solid solution.

Various techniques exist for preparing the solid dispersions of the invention including melt-extrusion (e.g. hot melt extrusion), spray-drying and solution-evaporation, in particular hot melt-extrusion and spray-drying, spray-drying being preferred.

The particles according to the invention can be prepared by first preparing a solid dispersion of the components, and then optionally grinding or milling said dispersion.

The melt-extrusion process comprises the following steps :
a) mixing ARN-509 and a poly(meth)acrylate copolymer,
b) optionally blending additives with the thus obtained mixture,
c) heating the thus obtained blend until one obtains a homogenous melt,
d) forcing the thus obtained melt through one or more nozzles; and
e) cooling the melt till it solidifies.

The terms "melt" and "melting" do not only mean the alteration from a solid state to a liquid state, but can also refer to a transition to a glassy state or a rubbery state, and in which it is possible for one component of the mixture to get embedded more or less homogeneously into the other. In particular cases, one component will melt and the other component(s) will dissolve in the melt thus forming a solution, which upon cooling may form a solid solution having advantageous dissolution properties.

One important parameter of melt extrusion is the temperature at which the melt-extruder is operating. For the melt extrusion process of the present invention, the operating temperature preferably ranges between about 160°C and about 190°C, more preferably ranges between about 160°C and 175°C. The lower temperature limit is defined by the point at which ARN-509 is still melting during extrusion with a given set of extrusion conditions. When ARN-509 is not completely molten, the extrudate may not provide the desired bioavailability. When the viscosity of the mixture is too high, the process of melt extrusion will be difficult. At higher temperatures the components may decompose to an unacceptable level. A person skilled in the art will recognize the most appropriate temperature range to be used.

The throughput rate is also of importance because the components may start to decompose when they remain too long in contact with the heating element.

It will be appreciated that the person skilled in the art will be able to optimize the parameters of the melt extrusion process within the above given ranges. The working temperatures will also be determined by the kind of extruder or the kind of configuration within the extruder that is used. Most of the energy needed to melt, mix and dissolve the components in the extruder can be provided by the heating elements. However, the friction of the material within the extruder may also provide a substantial amount of energy to the mixture and aid in the formation of a homogenous melt of the components.

A person skilled in the art will recognize the most appropriate extruder, such as, for example, a single screw, a twin screw extruder or a multi-screw extruder, for the preparation of the subject-matter of the present invention.

Spray-drying of a mixture of the components in a suitable solvent also yields a solid dispersion of said components or particles comprising or consisting of a solid dispersion of said components and may be a useful alternative to the melt-extrusion process, particularly in those cases where the poly(meth)acrylate copolymer is not sufficiently stable to withstand the extrusion conditions and where residual solvent can effectively be removed from the solid dispersion. Yet another possible preparation consists of preparing a mixture of the components in a suitable solvent, pouring said mixture onto a large surface so as to form a thin film, and evaporating the solvent therefrom.

Solvents suitable for spray-drying can be any organic solvent in which ARN-509 and the poly(meth)acrylate copolymer, in particular Eudragit® L 100-55 or Eudragit® E 100, are miscable. In an aspect of the invention, the boiling point of the solvent is lower than the Tg (glass transition temperature) of the solid dispersion. In addition, the solvent should have relatively low toxicity and be removed from the dispersion to a level that is acceptable according to The International Committee on Harmonization (ICH) guidelines. Removal of solvent to this level may require a post drying step such as for instance tray-drying, subsequent to the spray-drying process. Solvents include alcohols such as methanol, ethanol, n-propanol, iso-propanol, and butanol, in particular methanol; ketones such as acetone, methyl ethyl ketone and methyl iso-butyl ketone; esters such as ethyl acetate and propylacetate; and various other solvents such as acetonitrile, dichloromethane, toluene, and 1,1,1-trichloroethane. Lower volatility solvents such as dimethyl acetamide or dimethylsulfoxide can also be used. In an aspect of the invention, the solvent suitable for spray drying is a mixture of solvents. In an aspect of the invention the solvent for spray drying is a mixture of an alcohol and acetone, in particular a mixture of methanol and acetone, more in particular a mixture of methanol and acetone 1:9 (w:w). In an aspect of the invention the solvent for spray drying is a mixture of an alcohol and dichloromethane, in particular a mixture of methanol and dichloromethane, more in particular a mixture of methanol and dichloromethane 5:5 (w:w) or 6:4 (w:w), preferably 5:5 (w:w).

The particles as described herein have a d⁵⁰ of about 1500 µm, of about 1000 µm, of about 500 µm, of about 400 µm, of about 250 µm, of about 200µm, of about 150µm, of about 125 µm, of about 100µm, of about 70 µm, of about 65µm, of about 60µm, of about 55µm, of about 50µm, of about 45µm, of about 40µm, of about 35µm, of about 30µm, of about 25µm, or of about 20µm. Particles obtained by spray drying have preferably a d⁵⁰-value falling in the range from about 20µm to about 100µm, in particular a d⁵⁰-value falling in the range from about 20µm to about 70µm, more in particular a d⁵⁰-value of about 20µm, of about 25µm, of about 30µm, of about 35µm, of about 40µm, of about 45µm, of about 50µm, of about 55µm, of about 60µm, of about 65µm, or of about 70µm.

As used herein, the term d⁵⁰ has its conventional meaning as known to the person skilled in the art and can be measured by art-known particle size measuring techniques such as, for example, sedimentation field flow fractionation, photon correlation spectroscopy, laser diffraction or disk centrifugation. The d⁵⁰ mentioned herein may be related to volume distributions of the particles. In that instance, by "a d⁵⁰ of 50 µm" it is meant that at least 50% of the volume of the particles has a particle size of less than 50 µm. The same applies to the other particle sizes mentioned. In a similar manner, the d⁵⁰ particle size may be related to weight distributions of the particles. In that instance, by "d⁵⁰ of 50 µm" it is meant that at least 50% of the weight of the particles has a particle size of less than 50 µm. The same applies to the other particle sizes mentioned. Usually volume and weight distribution result in the same or about the same value for the average particle size.

The particle size can be an important factor determining the tabletting speed, in particular the flowability and therefore the manufacturability on a large scale of a particular dosage form or formulation, and the quality of the final product. For instance, for capsules, the particle size may range preferably from about 100 to about 1500 µm (d⁵⁰); for tablets the particle size is preferably less than 250 µm, more preferably less than 100 µm (d⁵⁰). Too small particles (< 10-20 µm) often cause sticking on the tablet punches and manufacturability issues.

The particles or solid dispersions as described herein may further comprise one or more pharmaceutically acceptable excipients such as, for example, plasticizers, flavors, colorants, preservatives and the like. Especially in case of preparation by hot melt extrusion, said excipients should not be heat-sensitive, in other words, they should not show any appreciable degradation or decomposition at the working temperature of the melt-extruder.

Suitable plasticizers are pharmaceutically acceptable and include low molecular weight polyalcohols such as ethylene glycol, propylene glycol, 1,2 butylene glycol, 2,3-butylene glycol, styrene glycol; polyethylene glycols such as diethylene glycol, triethylene glycol, tetraethylene glycol; other polyethylene glycols having a molecular weight lower than 1,000 g/mol; polypropylene glycols having a molecular weight lower than 200 g/mol; glycol ethers such as monopropylene glycol monoisopropyl ether; propylene glycol monoethyl ether; diethylene glycol monoethyl ether; ester type plasticizers such as triethyl citrate, sorbitol lactate, ethyl lactate, butyl lactate, ethyl glycolate, allyl glycollate; and amines such as monoethanolamine, diethanolamine, triethanolamine, monoisopropanolamine; triethylenetetramine, 2-amino-2-methyl-1,3-propanediol and the like. Of these, the low molecular weight polyethylene glycols, ethylene glycol, low molecular weight polypropylene glycols and especially propylene glycol are preferred.
In an aspect of the invention, the particles or solid dispersions as described herein do not contain a plasticizer.

The solid dispersions or the particles of the present invention can be formulated into pharmaceutical formulations comprising a therapeutically effective amount of ARN-509. Although, at first instance, pharmaceutical formulations for oral administration such as tablets and capsules are envisaged, the solid dispersions or the particles of the present invention can also be used to prepare pharmaceutical formulations e.g. for rectal administration. Preferred formulations are those adapted for oral administration shaped as a tablet. They can be produced by conventional tabletting techniques with conventional ingredients or excipients (pharmaceutically acceptable carrier) and with conventional tabletting machines. In order to facilitate the swallowing of such a formulation by a mammal, it is advantageous to give the formulations, in particular tablets, an appropriate shape. A film coat on the tablet may further contribute to the ease with which it can be swallowed.

The formulations of the invention, in particular the tablets, may include one or more conventional excipients (pharmaceutically acceptable carrier) such as disintegrants, diluents, fillers, binders, buffering agents, lubricants, glidants, thickening agents, sweetening agents, flavors, and colors. Some excipients can serve multiple purposes.

Preferably, the formulations of the present invention include a disintegrant, a diluent or filler, a lubricant and glidant.

Suitable disintegrants are those that have a large coefficient of expansion. Examples thereof are hydrophilic, insoluble or poorly water-soluble crosslinked polymers such as crospovidone (crosslinked polyvinylpyrrolidone) and croscarmellose sodium (crosslinked sodium carboxymethylcellulose). The amount of disintegrant in the tablets according to the present invention may conveniently range from about 3 to about 15 % (w/w) and preferably range from about 3 to 7 %, in particular is about 5 % (w/w). Because disintegrants by their nature yield sustained release formulations when employed in bulk, it is advantageous to dilute them with an inert substance called a diluent or filler.

A variety of materials may be used as diluents or fillers. Examples are lactose monohydrate, anhydrous lactose, sucrose, dextrose, mannitol, sorbitol, starch, cellulose (e.g. micro-crystalline cellulose (Avicel™), silicified microcrystalline cellulose), dihydrated or anhydrous dibasic calcium phosphate, and others known in the art, and mixtures thereof (e.g. spray-dried mixture of lactose monohydrate (75 %) with microcrystalline cellulose (25 %) which is commercially availble as Microcelac™). Preferred is microcrystalline cellulose and silicified microcrystalline cellulose. The amount of diluent or filler in the tablets may conveniently range from about 20 % to about 70 % (w/w) and preferably ranges from about 55 % to about 60 % (w/w).

Lubricants and glidants can be employed in the manufacture of certain dosage forms, and will usually be employed when producing tablets. Examples of lubricants and glidants are hydrogenated vegetable oils, e.g hydrogenated Cottonseed oil, magnesium stearate, stearic acid, sodium lauryl sulfate, magnesium lauryl sulfate, colloidal silica, colloidal anhydrous silica talc, mixtures thereof, and others known in the art. Interesting lubricants are magnesium stearate, and mixtures of magnesium stearate with colloidal silica. A preferred lubricant is magnesium stearate. A preferred glidant is colloidal anhydrous silica.
Glidants generally comprise 0.2 to 7.0 % of the total tablet weight, in particular 0.5 to 1.5%, more in particular 1 to 1.5% (w/w).
Lubricants generally comprise 0.2 to 7.0 % of the total tablet weight, in particular 0.2 to 1%, more in particular 0.5 to 1% (w/w).

Other excipients such as coloring agents and pigments may also be added to the formulations of the invention. Coloring agents and pigments include titanium dioxide and dyes suitable for food. A coloring agent is an optional ingredient in the formulation of the invention, but when used the coloring agent can be present in an amount up to 3.5 % based on the total tablet weight.

Flavors are optional in the formulation and may be chosen from synthetic flavor oils and flavoring aromatics or natural oils, extracts from plants leaves, flowers, fruits and so forth and combinations thereof. These may include cinnamon oil, oil of wintergreen, peppermint oils, bay oil, anise oil, eucalyptus, thyme oil. Also useful as flavors are vanilla, citrus oil, including lemon, orange, grape, lime and grapefruit, and fruit essences, including apple, banana, pear, peach, strawberry, raspberry, cherry, plum, pineapple, apricot and so forth, The amount of flavor may depend on a number of factors including the organoleptic effect desired. Generally the flavor will be present in an amount from about 0 % to about 3 % (w/w).

As known in the art, tablet blends may be dry-granulated or wet-granulated before tabletting. The tabletting process itself is otherwise standard and readily practised by forming a tablet from desired blend or mixture of ingredients into the appropriate shape using a conventional tablet press.

Tablets of the present invention may further be film-coated e.g. to improve taste, to provide ease of swallowing and an elegant appearance. Many suitable polymeric film-coating materials are known in the art. A preferred film-coating material is Opadry II 85F210036 Green. Other suitable film-forming polymers also may be used herein, including, hydroxypropylcellulose, hydroxypropyl methylcellulose (HPMC), especially HPMC 2910 5 mPa.s, and acrylate-methacrylate copolymers. Besides a film-forming polymer, the film coat may further comprise a plasticizer (e.g. propylene glycol) and optionally a pigment (e.g. titanium dioxide). The film-coating suspension also may contain talc as an anti-adhesive. In tablets according to the invention, the film coat in terms of weight preferably accounts for about 3 % (w/w) or less of the total tablet weight.

Preferred formulations are those wherein the weight of the particles or solid dispersions as described herein ranges from 20 to 40 %, in particular from 30 to 40 % of the total weight of the formulation.

The present invention further concerns a process of preparing solid dispersions as described herein, comprising blending ARN-509 and a poly(meth)acrylate copolymer and extruding said blend at a temperature in the range from about 160°C to about 190 °C.

The present invention further concerns a process of preparing particles as described herein, comprising blending ARN-509 and a poly(meth)acrylate copolymer, extruding said blend at a temperature in the range from about 160°C to about 190 °C, grinding the extrudate, and optionally sieving the particles.

Suitable extruders that may be used are the Haake mini-extruder, Leistritz 18 mm extruder, and the Leistritz 27 mm extruder.

The present invention further concerns a process of preparing particles or solid dispersions as described herein comprising mixing ARN-509 and a poly(meth)acrylate copolymer in a suitable solvent and spray drying said mixture. In an aspect, the suitable solvent is a mixture of dichloromethane and methanol. In an aspect, the suitable solvent is a mixture of dichloromethane and methanol wherein the w:w ratio of dichloromethane to methanol in the mixture is 4 : 6 or 5:5, preferably 5:5.

A preferred crystalline form of ARN-509 for preparing the solid dispersions or particles as described herein is Form B, which is an anhydrous crystalline form (see hereinafter and reference is also made to WO2013/184681, which is incorporated herein by reference).

It is another object of the invention to provide a process of preparing a pharmaceutical formulation as described herein, in particular in the form of a tablet or a capsule, characterized by blending a therapeutically effective amount of a solid dispersion or particles as described herein, with a pharmaceutically acceptable carrier and compressing said blend into tablets or filling said blend in capsules.

Further, this invention concerns a solid dispersion or particles as described herein, for use in preparing a pharmaceutical formulation for administration, in particular oral administration, to a mammal, in particular a human, suffering from an androgen receptor (AR)-related disease or condition, in particular cancer, more in particular prostate cancer, including but not limited to castration-resistant prostate cancer, metastatic castration resistant prostate cancer, chemotherapy-naive metastatic castration resistant prostate cancer, biochemically relapsed hormone sensitive prostate cancer, or high-risk, non-metastatic castration-resistant prostate cancer.

The present invention also concerns the solid dispersion or particles as described herein, for use in the administration, in particular oral administration, to a mammal, in particular a human, suffering from an androgen receptor (AR)-related disease or condition, in particular cancer, more in particular prostate cancer, including but not limited to castration-resistant prostate cancer, metastatic castration resistant prostate cancer, chemotherapy-naive metastatic castration resistant prostate cancer, biochemically relapsed hormone sensitive prostate cancer, or high-risk, non-metastatic castration-resistant prostate cancer.

The invention also relates to a pharmaceutical formulation as described herein for use in a method of treating an androgen receptor (AR)-related disease or condition, in particular cancer, more in particular prostate cancer, including but not limited to castration-resistant prostate cancer, metastatic castration resistant prostate cancer, chemotherapy-naive metastatic castration resistant prostate cancer, biochemically relapsed hormone sensitive prostate cancer, or high-risk, non-metastatic castration-resistant prostate cancer, in a mammal, in particular a human, which comprises administering, in particular orally, to said mammal, in particular human, an effective anticancer amount of a pharmaceutical formulation as described herein.

The invention further concerns the pharmaceutical formulation as specified herein, for use in treating an androgen receptor (AR)-related disease or condition, in particular cancer, more in particular prostate cancer, including but not limited to castration-resistant prostate cancer, metastatic castration resistant prostate cancer, chemotherapy-naive metastatic castration resistant prostate cancer, biochemically relapsed hormone sensitive prostate cancer, or high-risk, non-metastatic castration-resistant prostate cancer. Or, alternatively, the invention concerns a pharmaceutical formulation as specified herein for use in the treatment of an androgen receptor (AR)-related disease or condition, in particular cancer, more in particular prostate cancer, including but not limited to castration-resistant prostate cancer, metastatic castration resistant prostate cancer, chemotherapy-naive metastatic castration resistant prostate cancer, biochemically relapsed hormone sensitive prostate cancer, or high-risk, non-metastatic castration-resistant prostate cancer.

The invention also relates to a pharmaceutical package suitable for commercial sale comprising a container, a pharmaceutical formulation as described herein, and associated with said package written matter.

The term "about" as used herein in connection with a numerical value is meant to have its usual meaning in the context of the numerical value. Where necessary the word "about" may be replaced by the numerical value ±10%, or ±5%, or ±2%, or ±1%. All documents cited herein are incorporated by reference in their entirety.

The following examples are intended to illustrate the present invention.

### Example 1 : ARN-509 forms

For the preparation of different (crystalline) forms of ARN-509, reference is made to WO2013/184681. Different (crystalline or amorphous) forms of ARN-509 can be used to prepare the solid dispersions, particles or formulations according to the present invention.
A preferred form of ARN-509 for use in the preparation of the solid dispersions, particles or formulations according to the present invention is ARN-509 Form B, which is an anhydrous crystal. It was prepared by suspending ARN-509 Form A (reference is made to WO2013/184681, including for the diffraction data) in USP water and heating the slurry to 55±5 °C, holding at said temperature for at least 24 hours, followed by cooling the slurry to 25±5 °C. The resulting slurry was filtered, and the wet cake washed once with USP water. The wet cake was unloaded from the filter and dried under vacuum to afford ARN-509 Form B. Reference is also made to Example 2 below.
Solubility of Form A : 0.01 mg/ml in water.
Solubility of Form B : 0.004 mg/ml in water.

### Example 2

### Characterisation of ARN-509 Form B

### Powder XRD

X-ray powder diffraction (XRPD) analyses were carried out on a PANalytical (Philips) X'PertPRO MPD diffractometer. The instrument is equipped with a Cu LFF X-ray tube.
The compound was spread on a zero background sample holder.

### INSTRUMENT PARAMETERS

| | |
|---|---|
| generator voltage: | 45 kV |
| generator amperage: | 40 mA |
| geometry: | Bragg-Brentano |
| stage: | spinner stage |

### MEASUREMENT CONDITIONS

| scan mode: | continuous |
|---|---|
| scan range: | 3 to 50° 2θ |
| step size: | 0.02°/step |
| counting time: | 30 sec/step |
| spinner revolution time: | 1 sec |
| radiation type: | CuKα |

| ***Incident beam path*** | | ***Diffracted beam path*** | |
|---|---|---|---|
| program. divergence slit: | 15 mm | long anti scatter shield: | + |
| Soller slit: | 0.04 rad | Soller slit: | 0.04 rad |
| beam mask: | 15 mm | Ni filter: | + |
| anti scatter slit: | 1° | detector: | X'Celerator |
| beam knife: | + | | |

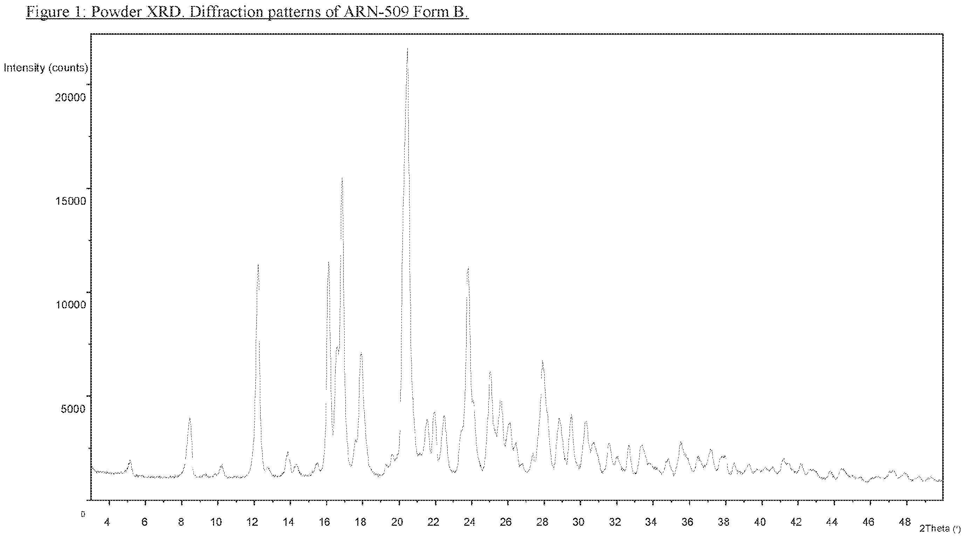

The X-ray powder diffraction pattern of ARN-509 Form B shows diffraction peaks without the presence of a halo, indicating that this compound is present as a crystalline product. The XRD pattern of ARN-509 Form B is shown in Figure 1.

### Infrared spectrometry (Micro ATR-IR)

The samples were analyzed using a suitable microATR accessory.

| | |
|---|---|
| apparatus: | Thermo Nexus 670 FTIR spectrometer |
| number of scans: | 32 |
| resolution: | 1 cm⁻¹ |
| wavelength range: | 4000 to 400 cm⁻¹ |
| detector: | DTGS with KBr windows |
| beamsplitter: | Ge on KBr |
| micro ATR accessory: | Harrick Split Pea with Si crystal |

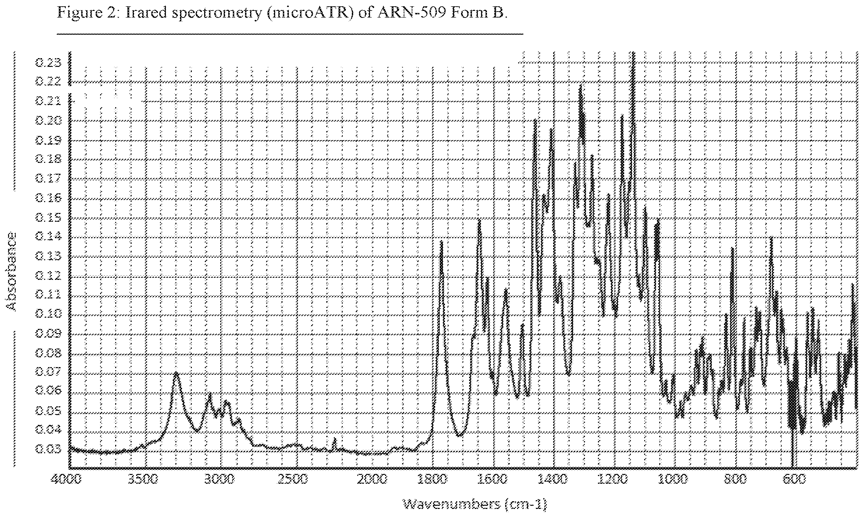

The spectrum of ARN-509 Form B is shown in Figure 2.

### Differential scanning calorimetry (DSC)

The compound was transferred into a standard aluminum TA-Instrument sample pan. The sample pan was closed with the appropriate cover and the DSC curve was recorded on a TA-Instruments Q1000 MTDSC equipped with a RCS cooling unit, using the following parameters:

| | |
|---|---|
| initial temperature: | 25°C |
| heating rate: | 10°C/min |
| final temperature: | 250°C |

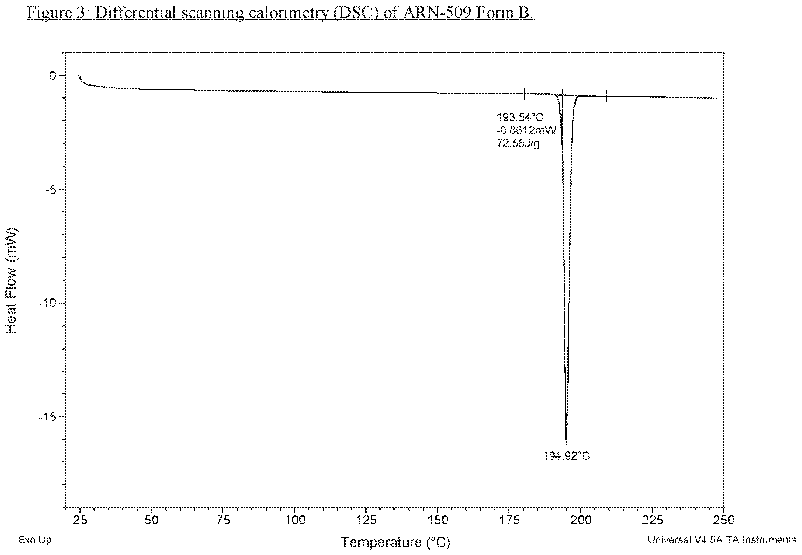

The DSC curve of ARN-509 Form B shows the melting of the product at 194.9°C with a heat of fusion of 73J/g. See Figure 3.

### Example 3.1 : Preparation of a solid dispersion of ARN-509: Eudragit® L 100-55 1:2

| | |
|---|---|
| ARN-509 | 333.33 mg |
| Eudragit® L 100-55 | 666.67 mg |
| Methanol | 1900.00mg |
| Acetone ^{a} | 17100.00 mg |

| | |
|---|---|
| ^{a} Removed during processing (the reported amounts are for 1 g of SDP (spray dried product) | |

The acetone and methanol were transferred into a suitable container, and Eudragit® L 100-55 and ARN-509 Form B, were added. After mixing the ingredients using a suitable mixer, the mixture was spray dried using a suitable spray dryer e.g. Buchi mini spray dryer with the following parameters : spray rate in the range from 6,4 - 6,7 gram/minutes, outlet temperature in the range from 49°C - 50°C and condenser temperature in the range from -18°C to -22°C. The spray dried product (SDP) was dried in a suitable dryer, e.g. tray dryer using vacuum, nitrogen flow and a drying temperature of 25°C.

### Example 3.2 : Preparation of tablets comprising a solid dispersion of ARN-509: Eudragit® L 100-55 1:2

| | |
|---|---|
| Spray dried powder of 3.1 (SDP) | 180.0 mg |
| Colloidal Anhydrous Silica | 9.1 mg |
| Croscarmellose sodium | 35.0 mg |
| Silicified Microcrystalline Cellulose | 472.4 mg |
| Magnesium stearate | 3.5 mg |
| | (amounts for 1 tablet) |

| | |
|---|---|
| Spray dried powder of 3.1 (SDP) | 360.0 mg |
| Colloidal Anhydrous Silica | 18.2 mg |
| Croscarmellose sodium | 70.0 mg |
| Silicified Microcrystalline Cellulose | 944.8 mg |
| Magnesium stearate | 7.0 mg |
| (amounts for 1 tablet) | |

The SDP, part (3555/4724) of the silicified microcrystalline cellulose, part (10/13) of the colloidal anhydrous silica and part (1/2) of the croscarmellose sodium were sieved and mixed to a homogenous blend using a suitable blender. A dry granulate was made by using a suitable compaction technique. The remainder of the silicified microcrystalline cellulose (1169/4724), colloidal anhydrous silica (3/13) and croscarmellose sodium (1/2) were sieved and added to the dry granulate and further mixed using a suitable blender. Magnesium stearate was sieved and added to the blend and mixed further using a suitable blender. The blend was compressed into tablets using an eccentric tabletting press.

### Example 4.1 : Preparation of a solid dispersion of ARN-509: Eudragit® L 100-55 1:2 by hot melt extrusion (HME)

| | |
|---|---|
| ARN-509 | 333.33 mg |
| Eudragit® L 100-55 | 666.67 mg |
| (the reported amounts are for 1 g of HME product) | |

The Eudragit® L 100-55 and ARN-509 Form B, were blended in a suitable recipient using a suitable blender. Hot melt extrusion was performed in a Haake extruder, flush mode, maximum temperature 190 °C, screw speed 30 rpm. The hot melt extrudate was collected and milled in a suitable mill. The milled hot melt extrudate was sieved using a suitable sieve (250 µm).

### Example 4.2 : Preparation of tablets comprising a solid dispersion of ARN-509: Eudragit® L 100-55 1:2 (HME)

| | |
|---|---|
| HME powder of 4.1 | 180.0 mg |
| Colloidal Anhydrous Silica | 9.1 mg |
| Croscarmellose sodium | 35.0 mg |
| Silicified Microcrystalline Cellulose | 472.4 mg |
| Magnesium stearate | 3.5 mg |
| (amounts for 1 tablet) | |

The silicified microcrystalline cellulose, croscarmellose sodium and colloidal anhydrous silica were sieved and mixed with the hot melt extrudate to a homogenous blend using a suitable blender. Magnesium stearate was sieved and added to the blend and mixed further using a suitable blender. The blend was compressed into tablets using an eccentric tableting press.

### Example 5

### Bioavailability study

### Test system

Species: Marshall beagle dogs
Supplier: Marshall Farms, Italy/USA
Gender and age: male (n = 12), approximately 1-7 years
Body weights: 8-13 kg at the start of the experimental phase

### Diet and water supply:

Continuous access to water
Dosing : fasted for about 21 hours before dosing till ± 2 hours after dosing.
Afterwards, dogs had free access to food until the late afternoon.

### Test compound and formulations

Formulation 1: 60 mg tablet containing ARN-509- Eudragit® L 100-55 ratio 1/2 SDP
Formulation 2: 60 mg tablet containing ARN-509- Eudragit® L 100-55 ratio 1/2 HME
Formulation 3: a nonaqueous, lipid-based solution filled into softgel capsules, each containing 30 mg ARN-509

### Blood sampling and plasma preparation

Blood samples (2 ml on EDTA) were taken from a jugular vein. Within 1 hour of sampling, the blood samples were centrifuged and within 2 hours after the start of centrifugation, plasma was stored in the freezer.

### Dose administration

| **Day of Dosing** | **Formulation** | **Route** | **Dose (mg/dog)** | **Tablet (pcs)** | **Dog Nos.** |
|---|---|---|---|---|---|
| Day 0 | **Formulation 1** | PO (gavage) | 60 | 1 | n=4 |
| | **Formulation 2** | PO (gavage) | 60 | 1 | n=4 |
| | **Formulation 3** | PO (gavage) | 60 | 2 | n=4 |

### Bioanalysis

All study samples were analyzed using a qualified LC-MS/MS method. The samples were subjected to a selective sample cleanup, followed by HPLC-MS/MS.
HPLC separation was done using non-chiral reversed phase liquid chromatography. Subsequent MS/MS analysis was performed using triple quadrupole mass spectrometry in the Multiple Reaction Monitoring (MRM) mode, optimized for the compound. Samples were quantified against calibration curves prepared to cover the concentration range of the study samples. The curves were prepared in the same matrix as the study samples. For each analytical batch, independent quality control samples, prepared in the same matrix as the samples, were analyzed together with the study samples and calibration curve. All analytical batches were accepted based on calibration curve and QC acceptance criteria in line with the current FDA guidelines.

### Data analysis

Individual plasma concentration-time profiles were subjected to a pharmacokinetic analysis using validated Phoenix software. A non-compartmental analysis using the linear up/log down trapezoidal rule was used for all data.

### Results

The mean Cₘₐₓ, Tₘₐₓ, AUC and Fᵣₑₗ values of ARN-509 in male beagle dogs after single oral administration of the 3 formulations are presented below:

| **Formulation** | **1** | **2** | **3** |
|---|---|---|---|
| Cmax (ng/ml) | 4210 | 2860 | 4110 |
| Tₘₐₓ (h) | 1-2 | 0.5-24 | 1-2 |
| AUCₗₐₛₜ (ng.h/ml) ¹⁾ | 190000 | 149000 | 157000 |
| AUC_{0-inf} (ng.h/ml) | 105000²⁾ | 94600²⁾ | 167000 |
| Frel (AUCₗₐₛₜ ratios) | 121% | 95% | |

| | | | |
|---|---|---|---|
| ¹⁾ Tₗₐₛₜ in majority of animals was 168h; ²⁾ n=2 | | | |

Formulation 1: Eudragit® L 100-55 ratio 1/2 (SDP)
Formulation 2: Eudragit® L 100-55 ratio 1/2 (HME)
Formulation 3: softgel reference capsule

### Example 6 : Preparation of a solid dispersion of ARN-509: Eudragit® L 100-55 1:2 (SDP)

| | |
|---|---|
| ARN-509 | 333.33 mg |
| Eudragit® L 100-55 | 666.67 mg |
| Methanol ^{a} | 9500.00 mg |
| Dichloromethane ^{a} | 9500.00 mg |

| | |
|---|---|
| ^{a} Removed during processing (the reported amounts are for 1 g of SDP (spray dried product) | |

The dichloromethane and methanol were transferred into a suitable container and stirring was started. Under continuous stirring ARN-509 Form B was added to the solvent mixture and stirred until dissolved. Eudragit® L 100-55 was added to the solution and stirred overnight. A clear solution was obtained. The solution was filtered inline through a GRID filter. The solution was spray dried using a suitable spray dryer, e.g. Niro A/S PSD3 with a high pressure nozzle with the following parameters : feed flow of 75 kg/hour, outlet temperature of 40 °C and a condenser temperature of -8 °C. The spray dried product (SDP) was dried in a suitable dryer, e.g. tray dryer using vacuum, nitrogen flow and a drying temperature of 50 °C.

### Stability tests performed on spray-dried powder

The below stability tests were performed on the powder of Example 6 packed in LDPE/Alu bags.

### 1.Appearance testing

A visual examination was performed on the powder stored under different storage conditions as indicated in table a below.

The results are reported in table a below.

### 2.Water content

The water content was determined by means of a vaporized coulometric Karl Fischer determination in accordance with USP/Ph. Eur.

The powder was stored as indicated in table a below.

About 50.00 mg (±5.00 mg) of the sample was weighted accurately into a vial and the vial was crimped securely.

The results are reported in table a below.

The following instrumentation, reagents and solutions and parameters were used.

### INSTRUMENTATION

Coulometer: 831 KF Coulometer Metrohm
Oven: 774 Sample Oven Processor Metrohm
Generator electrode: Electrode with diapraghm Metrohm 6.0344.100
Indicator electrode: Double Pt-wire electrode Metrohm 6.0341.100

### REAGENTS AND SOLUTIONS

Anode solution: Hydranal Coulomat AG Oven (Fluka 34739)
Cathode solution: Hydranal Coulomat CG (Fluka 34840)
Water standard: Hydranal Water Standard 1.00 (Fluka 34828)

### Oven Parameters

Carrier gas : N₂
Flow rate : Setpoint 60 mL/min
Read out value minimum 20 mL/min
Oven temperature : 120°C

### Coulometer Parameters

Titration Parameters
Extr. time : 60 s
Drift correction : Auto
Start Conditions
Pause : 60 s
Start drift: maximum 12 µg/min
Time cond. OK : 10 s
Stop Parameters
Rel. drift: 5 µg/min

Alternative coulometer parameters may be used provided system suitability requirements are met

### 3. pXRD testing for the detection of crystalline ARN-509

The physical stability of the powder stored under different storage conditions was followed up using powder X-Ray diffraction. The XRD pattern of the powder was compared to the XRD pattern of the corresponding powder measured at time zero (amorphous product).

The powder was brought on to the zero background sample holder. A X-ray measurement of the sample was performed.

The results are reported in table a below.

The following instrumentation and parameters were used.

### INSTRUMENTATION

Pananalytical X'Pert PRO MPD diffractometer PW3050/60
X-ray tube Cu LFF PW3373/10
Detector: X'Celerator
Sample stage: spinner
Sample holder: zero background sample holder

### Instrument settings

Spinner revolution time: 1 rps
Generator voltage: 45 kV
Generator current: 40 mA

### Optical components in X-ray beam path

### Incident beam path:

Programmable divergence slit: irradiated length 15 mm
Soller slit: 0.04 rad
Beam mask: 15 mm
Anti-scatter slit: 1°
Beam knife +

### Diffracted beam path:

Programmable Anti-scatter slit: 1°
Soller slit: 0.04 rad
Filter: Ni

### INSTRUMENT PARAMETERS

Geometry: Bragg-Brentano
Radiation: CuKα
Step size: 0.02°
Scan range: from 3° 2θ to 50° 2θ
Counting time per step:60 sec

**Table a: Test conditions and results for the powder stored in LDPE/Alu Bags - appearance and water content and crystallinity results**

| Parameter | | Appearance ^{a} | Water content (%) | Crystallinity |
|---|---|---|---|---|
| Storage condition | Storage time (months) | Visual examination | | |
| | Initial | Pass | 0.3 | Amorphous product |
| 30°C/75%RH | 6 | Pass | 1.1 | Amorphous product |

| | | | | |
|---|---|---|---|---|
| ^{a} Pass : White to light yellow, fine to granular powder | | | | |

For the appearance, no substantial stability related changes were observed during storage of the drug product intermediate powder at the different storage conditions. For the water content, no substantial stability related changes were observed during storage of the drug product intermediate powder at the different storage conditions.

For crystallinity, no substantial stability related changes were observed during storage of the drug product at the different storage conditions.

### 4. Assay of ARN-509-chromatographic purity

The concentration of ARN-509 and its degradation products in the powder stored under different storage conditions were determined by gradient Reversed-Phase UHPLC with UV Detection.

Powders were stored as indicated in table b below.

180.00 mg powder was weighted accurately into a 250-mL volumetric flask. Approximately 125 mL acetonitrile was added by graduated cylinder and the whole was shaken mechanically for 30 minutes and diluted to volume with water till approximately 1 cm under the marker. The whole was shaked up manually vigorously. The sample solution was allowed to equilibrate to ambient temperature and was diluted to volume with water. Just before filtering, the volumetric flask was shaked up manually vigorously. The sample solution was filtered through a chemical resistant 0.2 µm filter. The first 3 mL filtrate was discarded into a waste container, not back into the volumetric flask.
The sample solution is stable for 4 days, if stored in refrigerator, protected from light (closed cabinet).

The results are reported in table b below.

The following solutions and instrumentation and parameters were used.

### Mobile Phases

**Mobile Phase A**
   10 mM NH₄Ac + 0.1% TFA / Acetonitrile (90/10, v/v).
**Mobile Phase B**
   Acetonitrile

### UHPLC Conditions for Identification, Assay and Chromatographic Purity

Column: Acquity BEH C18, 150 mm length × 2.1 mm i.d., 1.7 µm particle size
Column Temperature: 45 °C
Auto-Sampler Temperature: 5 °C
Flow Rate: 0.40 mL/min
Detection: UV
Wavelength: 268 nm
Injection Volume: 3 µL
Data Collection Time: 35 minutes
Analysis Run Time: 40 minutes

A linear gradient was programmed as demonstrated in the below table.

### Linear Gradient Program

| Time (min) | A (% vol) | B (% vol) |
|---|---|---|
| 0 | 100 | 0 |
| 35 | 30 | 70 |
| 36 | 100 | 0 |
| 40 | 100 | 0 |

**Table b: Test conditions and results for the powder stored in LDPE/Alu Bags-assay and degradation products results**

| Parameter | | Assay(%) | Degradation products (%) |
|---|---|---|---|
| Storage condition | Storage time (months) | ARN-509 | Total degradation products (sum of all degradation products ≥0.05% (totals are calculated on unrounded results) |
| | Initial | 102.3 | 0.11 |
| 30°C/75%RH | 6 | 101.7 | 0.12 |

No substantial stability related changes were observed during storage of the drug product intermediate powders at the different storage conditions.

### 5. Water activity

The water activity was determined with a Novasina a_{w}-meter.

The results are reported in table c below.

**Table c: Test condition and result for the powder -Water activity results**

| Parameter | | Water activity |
|---|---|---|
| Storage condition | Storage time (months) | |
| | Initial | 0.13 |

### Stability tests performed on coated tablets prepared from spray-dried powder

The following tablets were prepared from the powder of Example 6 analogous to Example 3.2.

| | |
|---|---|
| Spray dried powder (SDP) | 180 mg |
| Colloidal Anhydrous Silica | 7 mg |
| Croscarmellose sodium | 17.5 mg |
| Microcrystalline Cellulose | 355.5 mg |
| Colloidal Anhydrous Silica | 2.1 mg |
| Croscarmellose sodium | 17.5 mg |
| Silicified Microcrystalline Cellulose | 116.9 mg |
| Magnesium stearate (amounts for 1 tablet) | 3.5 mg |

The tablets were coated with green OPADRY II 85F210036.

| | |
|---|---|
| ARN-509 (60 mg) Tablets | 600.18g |
| Opadry II 85F210036 Green | 22.53g |
| Purified Water ^{a} | 89.97g |

| | |
|---|---|
| ^{a} Removed during processing per batch of 857 tablets | |

The purified water was transferred into a suitable container. The coating powder was added and mixed with a suitable mixer. The core tablets were film coated with the coating suspension using a suitable coater.

The stability tests were performed on the above tablets stored in HDPE Bottles with Desiccant (silica gel).

### 1.Appearance testing

A visual examination was performed on the tablets stored under different storage conditions as indicated in table 1 below.

The results are reported in table 1 below.

### 2.Water content

The water content was determined by means of a vaporized coulometric Karl Fischer determination in accordance with USP/Ph. Eur.

Tablets were stored as indicated in table 1 below.

Tablets were grinded using a Retsch Mixer Mill. Immediately after grinding, about 50.00 mg (±5.00 mg) of the sample was weighted accurately into a vial and the vial was crimped securely.

The results are reported in table 1 below.

The following instrumentation, reagents and solutions and parameters were used.

### INSTRUMENTATION

Coulometer: 831 KF Coulometer Metrohm
Oven: 774 Sample Oven Processor Metrohm
Generator electrode: Electrode with diapraghm Metrohm 6.0344.100
Indicator electrode: Double Pt-wire electrode Metrohm 6.0341.100

### REAGENTS AND SOLUTIONS

Anode solution: Hydranal Coulomat AG Oven (Fluka 34739)
Cathode solution: Hydranal Coulomat CG (Fluka 34840)
Water standard: Hydranal Water Standard 1.00 (Fluka 34828)

### Oven Parameters

Carrier gas : N₂
Flow rate : Setpoint 60 mL/min
Read out value minimum 20 mL/min
Oven temperature : 120°C

### Coulometer Parameters

Titration Parameters
Extr. time : 60 s
Drift correction : Auto
Start Conditions
Pause : 60 s
Start drift: maximum 12 µg/min
Time cond. OK : 10 s
Stop Parameters
Rel. drift: 5 µg/min

Alternative coulometer parameters may be used provided system suitability requirements are met

**Table 1: Test conditions and results for tablets stored in HDPE Bottles with Desiccant (silica gel) -appearance and water content results**

| Parameter | | Appearance ^{a} | Water content (%) |
|---|---|---|---|
| Storage condition | Storage time (months) | Visual examination | |
| | Initial | Pass | 3.4 |
| -20°C | 1 | Pass | 2.6 |
| 5°C | 3 | Pass | 3.5 |
| | 6 | Pass | 2.4 |
| 25°C/60%RH | 6 | Pass | 2.5 |
| 30°C/75%RH | 1 | Pass | 2.7 |
| | 3 | Pass | 3.6 |
| | 6 | Pass | 2.8 |
| 40°C/75%RH | 1 | Pass | 2.6 |
| | 3 | Pass | 3.5 |
| | 6 | Pass | 3.4 |
| 50°C | 1 | Pass | 2.3 |
| | 3 | Pass | 3.0 |
| Light ICH | | Pass | 2.7 |

| | | | |
|---|---|---|---|
| ^{a} Pass : Greenish colored, oblong tablet Light ICH: integrated near UV energy not less than 200 Wh/m², overall illumination not less than 1200 klux·h | | | |

For the appearance, no substantial stability related changes were observed during storage of the drug product at the different storage conditions.

### 3. pXRD testing for the detection of crystalline ARN-509

The physical stability of different tablets stored under different storage conditions was followed up using powder X-Ray diffraction. The XRD pattern of the tablets was compared to the XRD pattern of the corresponding tablets at time zero (amorphous product).

One tablet was gently grinded to a fine powder using a mortar and pestle. The powder was loaded into the 16 mm sample holder using the back loading technique. A X-ray measurement of the sample was performed.

The results are reported in table 2 below.

The following instrumentation and parameters were used.

### INSTRUMENTATION

Philips X'Pert PRO MPD diffractometer PW3050/60
X-ray tube Cu LFF PW3373/10
Detector: X'Celerator
Sample stage: spinner
Sample holder: cavity diameter 16 mm, cavity depth 2.5 mm

### Instrument settings

Spinner revolution time: 1 rps
Generator voltage: 45 kV
Generator current: 40 mA

### Optical components in X-ray beam path

### Incident beam path:

Programmable divergence slit: irradiated length 10 mm
Soller slit: 0.04 rad
Beam mask: 10 mm
Anti-scatter slit: 1°
Beam knife +

### Diffracted beam path:

Programmable Anti-scatter slit: 1°
Soller slit: 0.04 rad
Filter: Ni

### INSTRUMENT PARAMETERS

Geometry: Bragg-Brentano
Radiation: CuKα
Step size: 0.02°
Scan range: from 3° 2θ to 50° 2θ
Counting time per step: 100 sec

**Table 2: Test conditions and results for tablets stored in HDPE Bottles with Desiccant (silica gel)-crystallinity results**

| Parameter | | Crystallinity |
|---|---|---|
| Storage condition | Storage time (months) | |
| | Initial | Amorphous drug substance in drug product |
| -20°C | 1 | Amorphous drug substance in drug product |
| 5°C | 3 | Amorphous drug substance in drug product |
| | 6 | Amorphous drug substance in drug product |
| 25°C/60%RH | 6 | Amorphous drug substance in drug product |
| 30°C/75%RH | 1 | Amorphous drug substance in drug product |
| | 3 | Amorphous drug substance in drug product |
| | 6 | Amorphous drug substance in drug product |
| 40°C/75%RH | 1 | Amorphous drug substance in drug product |
| | 3 | Amorphous drug substance in drug product |
| | 6 | Amorphous drug substance in drug product |
| 50°C | 1 | Amorphous drug substance in drug product |
| | 3 | Amorphous drug substance in drug product |
| Light ICH | | Amorphous drug substance in drug product |

| | | |
|---|---|---|
| Light ICH: integrated near UV energy not less than 200 W·h/m², overall illumination not less than 1200 klux·h | | |

No substantial stability related changes were observed during storage of the drug product at the different storage conditions.

### 4. Assay of ARN-509-chromatographic purity

The concentration of ARN-509 and its degradation products in tablets stored under different storage conditions were determined by gradient Reversed-Phase UHPLC with UV Detection.

Tablets were stored as indicated in table 3 below.

Five tablets were weighted accurately. Mean tablet weight was determined. Tablets were grinded to a fine powder. An amount of homogenized powder equivalent to the mean tablet weight was accurately weighted into a 250-mL volumetric flask. Approximately 125 mL acetonitrile was added by graduated cylinder and the whole was shaken mechanically for 30 minutes and diluted to volume with water till approximately 1 cm under the marker. The whole was shaked up manually vigorously. The sample solution was allowed to equilibrate to ambient temperature and was diluted to volume with water. Just before filtering, the volumetric flask was shaked up manually vigorously. The sample solution was filtered through a chemical resistant 0.2 µm filter. The first 3 mL filtrate was discarded into a waste container, not back into the volumetric flask.
The sample solution is stable for 4 days, if stored in refrigerator, protected from light (closed cabinet).

The results are reported in table 3 below.

The following solutions and instrumentation and parameters were used.

### Mobile Phases

**Mobile Phase A**
   10 mM NH₄Ac (aqueous ammonium acetate) + 0.1% TFA (trifluoroacetic acid) / Acetonitrile (90/10, v/v).
**Mobile Phase B**
   Acetonitrile

### UHPLC Conditions for Identification, Assay and Chromatographic Purity

Column: Acquity BEH C18, 150 mm length × 2.1 mm i.d., 1.7 µm particle size
Column Temperature: 45 °C
Auto-Sampler Temperature: 5 °C
Flow Rate: 0.40 mL/min
Detection: UV
Wavelength: 268 nm
Injection Volume: 3 µL
Data Collection Time: 35 minutes
Analysis Run Time: 40 minutes

A linear gradient was programmed as demonstrated in the below table.

### Linear Gradient Program

| Time (min) | A (% vol) | B (% vol) |
|---|---|---|
| 0 | 100 | 0 |
| 35 | 30 | 70 |
| 36 | 100 | 0 |
| 40 | 100 | 0 |

**Table 3: Test conditions and results for tablets stored in HDPE Bottles with Desiccant (silica gel)-assay and degradation products results**

| Parameter | | Assay(%) | Degradation products (%) |
|---|---|---|---|
| Storage condition | Storage time (months) | ARN-509 | Total degradation products (sum of all degradation products ≥0.05% (totals are calculated on unrounded results) |
| | Initial | 99.1 | 0.11 |
| -20°C | 1 | 99.2 | 0.07 |
| 5°C | 3 | 100.6 | 0.12 |
| | 6 | 100.7 | 0.11 |
| 25°C/60%RH | 6 | 101.0 | 0.11 |
| 30°C/75%RH | 1 | 100.8 | 0.11 |
| | 3 | 99.4 | 0.12 |
| | 6 | 99.4 | 0.07 |
| 40°C/75%RH | 1 | 98.2 | 0.06 |
| | 3 | 99.4 | 0.12 |
| | 6 | 99.4 | 0.07 |
| 50°C | 1 | 100.0 | 0.11 |
| | 3 | 99.3 | 0.12 |
| Light ICH | | 98.5 | 0.11 |

| | | | |
|---|---|---|---|
| Light ICH: integrated near UV energy not less than 200 Wh/m², overall illumination not less than 1200 klux·h | | | |

No substantial stability related changes were observed during storage of the drug product at the different storage conditions.

### 5.Dissolution

The dissolution test was performed using Paddle Apparatus (USP type 2, Ph.Eur., JP.) at 75 rpm in 900 mL of 0.5% (w/v) cetyltrimethylammonium bromide (CTAB) in 0.05 M sodium phosphate buffer pH 4.5.
Samples were taken by Distek® sample needles with solid housing and samples were filtered with Whatman®Spartan® 0.45 µm RC (regenerated cellulose) membrane 30 mm diameter filters.

The determination of the quantity of ARN-509 present in the dissolution samples was based upon an isocratic ultra high performance liquid chromatographic (UHPLC) method with UV detection.

The test was performed on tablets stored under different storage conditions as indicated in table 4 below.

The following instrumentation, reagents and solutions and parameters were used.

### INSTRUMENTATION

Dissolution Instrument: Paddle apparatus (USP type 2, Ph. Eur., JP).
UHPLC Instrument: Waters Acquity H-Class with UV detector.
Data Acquisition System: Waters Empower.
Analytical Balance: Sensitive to 0.01 g.
Analytical Balance: Sensitive to 0.01 mg.
pH Meter: Sensitive to 0.01 pH units.
Thermometer: Sensitive to 0.1 °C.

### REAGENTS AND SOLUTIONS

### Reagents

Cetrimonium bromide, cetyltrimethylammonium bromide,
hexadecyltrimethylammonium bromide,(CTAB): Pro Analysis, 99.0% Purity.
Sodium phosphate monobasic monohydrate (NaH₂PO₄.H₂O): ACS Grade.
Ammonium acetate: HPLC Grade, 99% Purity.
Acetonitrile: HPLC Grade.

### Mobile Phase

**Mobile Phase A:** 10 mM ammonium acetate
**Mobile Phase B:** Acetonitrile

### PROCEDURE

### Dissolution Parameters

Apparatus: Paddle Apparatus (USP type 2, Ph.Eur, JP.).
Vessels: 1-L glass.
Rotation Speed: 75 rpm.
Dissolution Medium: 0.5% (w/v) CTAB in 0.05 M Phosphate Buffer pH 4.5.
Volume of Medium: 900 mL.
Medium Degassing: Not Required.
Medium Replacement: Not Required.
Temperature: 37.0 ±0.5 °C.
Sinker: Use no sinker.
Sample Introduction: Transfer 1 tablet into each dissolution vessel.

### Analytical Finish - UHPLC Parameters

### Conditions

Column: Acquity UHPLC® BEH C18 1.7-µm particle size, 50 ×2.1 mm i.d.
Column Temperature: 45 ± 5 °C.
Sample Temperature: Ambient.
Flow Rate: 0.6 mL/min.
Detection: UV at 242 nm.
Injection Volume: 2 µL.
Elution Mode: Isocratic.
Mobile Phase: 50/50 (v:v), 10 mM Ammonium acetate : Acetonitrile.
Degas using suitable means.
Run Time (guide): 1.5 minutes.
Retention Time (guide): Approximately 0.7 minutes for ARN-509
Wash Solvent: Methanol.
Purge Solvent: 75/25 (v:v), Water/Methanol.
Sampling Rate: 20 points/sec with filter constant normal.

**Table 4: Test conditions and results for tablets stored in HDPE Bottles with Desiccant (silica gel)-Dissolution results**

| Parameter | | Dissolution mean (%)(min-max) | | | | | | |
|---|---|---|---|---|---|---|---|---|
| Storage condition | Storage time (months) | 5 min | 10 min | 15 min | 20 min | 30 min | 45 min | 60 min |
| | Initial | 53 (52-54) | 76 (75-76) | 87 (86-88) | 93 (92-95) | 97 (96-99) | 99 (98-101) | 100 (98-102) |
| -20°C | 1 | 56 (53-58) | 77 (77-78) | 87 (86-88) | 96 (93-98) | 97 (95-98) | 99 (96-100) | 99 (96-101) |
| 5°C | 3 | 57 (55-59) | 79 (78-79) | 88 (87-90) | 94 (91-96) | 98 (95-99) | 100 (97-102) | 100 (97-102) |
| | 6 | 54 (53-56) | 76 (76-78) | 85 (84-86) | 90 (89-92) | 94 (93-95) | 96 (94-97) | 96 (94-97) |
| 25°C/ 60%RH | 6 | 52 (50-55) | 75 (73-78) | 86 (83-88) | 92 (88-94) | 96 (93-99) | 98 (94-100) | 98 (94-100) |
| 30°C/ 75%RH | 1 | 54 (53-55) | 77 (75-78) | 87 (86-88) | 93 (91-94) | 97 (95-99) | 99 (97-101) | 99 (98-101) |
| | 3 | 56 (55-57) | 79 (78-79) | 89 (88-90) | 94 (94-95) | 99 (98-100) | 101 (100-102) | 101 (100-103) |
| | 6 | 52 (50-54) | 74 (73-75) | 85 (84-86) | 90 (89-91) | 95 (94-97) | 97 (95-99) | 97 (96-99) |
| 40°C/ 75%RH | 1 | 57 (55-62) | 78 (76-78) | 88 (86-89) | 93 (90-94) | 97 (94-100) | 99 (95-100) | 99 (96-100) |
| | 3 | 51 (50-52) | 73 (72-74) | 84 (83-86) | 90 (89-92) | 96 (94-97) | 98 (96-100) | 98 (96-100) |
| | 6 | 52 (50-53) | 74 (74-75) | 84 (82-86) | 90 (88-92) | 95 (93-97) | 97 (94-99) | 97 (95-100) |
| 50°C | 1 | 55 (54-55) | 77 (75-80) | 87 (86-88) | 93 (91-95) | 97 (95-100) | 99 (97-101) | 100 (98-103) |
| | 3 | 49 (49-51) | 73 (72-74) | 84 (83-85) | 90 (88-93) | 95 (93-98) | 97 (94-100) | 98 (94-101) |
| Light ICH | | 55 (54-56) | 77 (76-78) | 87 (86-88) | 92 (90-94) | 96 (94-99) | 98 (96-101) | 98 (96-101) |

| | | | | | | | | |
|---|---|---|---|---|---|---|---|---|
| Light ICH: integrated near UV energy not less than 200 W·h/m², overall illumination not less than 1200 klux·h | | | | | | | | |

No substantial stability related changes were observed during storage of the drug product at the different storage conditions.

### 6. Water activity

The water activity was determined with a Novasina a_{w}-meter.

The results are reported in table 5 below.

**Table 5: Test conditions and results for tablets-Water activity results**

| Parameter | | Water activity |
|---|---|---|
| Storage condition | Storage time (months) | |
| | Initial | 0.51 |

### 7. Microbiological purity

The microbiological purity of the tablets was tested according to USP <61> and <62>, and Ph.Eur.2.6.12 and 2.6.13.

Results are reported in table 6 below.

**Table 6: Test conditions and results for tablets of Example 3.3 stored in HDPE Bottles with Desiccant (silica gel)-Microbiological Purity Results**

| Parameter | | Total Aerobic Microbial count (cfu/g) | Total combined Molds and Yeasts Count (cfu/g) | Pathogens : E. Coli |
|---|---|---|---|---|
| Storage condition | Storage time (months) | USP <61>/ Ph.Eur.2.6.12 | USP <61>/ Ph.Eur.2.6.12 | USP <62>/ Ph.Eur.2.6.13 |
| | Initial | <50 | <50 | Absent in 1g |

No substantial stability related changes were observed during storage of the drug product at the different storage conditions.

It is within the knowledge of the skilled person to recognize equivalent conditions, solutions, reagents, parameters and instrumentation to the ones described above. It is within the knowledge of the skilled person to recognize appropriate reference solutions, calculation methods, suitability tests.

## Claims

1. A solid dispersion comprising 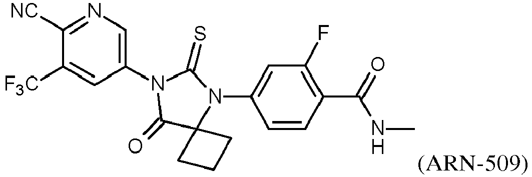 and a poly(meth)acrylate copolymer.

2. The solid dispersion according to claim 1 wherein the dispersion consists of ARN-509 and a poly(meth)acrylate copolymer.

3. The solid dispersion according to claim 1 or 2 wherein the weight-by-weight ratio of ARN-509 : poly(meth)acrylate copolymer in the solid dispersion is in the range from 1 : 1 to 1 : 5.

4. The solid dispersion according to claim 3 wherein the weight-by-weight ratio of ARN-509 : poly(meth)acrylate copolymer in the solid dispersion is 1:2.

5. The solid dispersion according to any one of the preceding claims wherein ARN-509 is present in amorphous form.

6. The solid dispersion according to any one of the preceding claims wherein the dispersion is a solid solution.

7. The solid dispersion according to any one of the preceding claims wherein the poly(meth)acrylate copolymer is poly(methacrylic acid-co-ethyl acrylate) 1:1.

8. The solid dispersion according to any one of the preceding claims obtainable by spray drying.

9. The solid dispersion according to any one of claims 1 to 7 obtainable by hot melt extrusion.

10. A particle consisting of a solid dispersion as defined in any one of the preceding claims.

11. A particle comprising a solid dispersion as defined in any one of claims 1 to 9.

12. A pharmaceutical formulation comprising a pharmaceutically acceptable carrier and a solid dispersion according to any one of claims 1 to 9.

13. A pharmaceutical formulation comprising a pharmaceutically acceptable carrier and a particle according to claim 10 or 11.

14. The formulation according to claim 12 or 13 wherein the formulation comprises 60 mg of ARN-509.

15. The formulation according to claim 12 or 13 wherein the formulation comprises 120 mg of ARN-509.

16. The formulation according to claim 12 or 13 wherein the formulation comprises 240 mg of ARN-509.

17. The formulation according to any one of claims 12 to 16 wherein the weight of the solid dispersion ranges from 20 to 40 % of the total weight of the formulation.

18. The formulation according to any one of claims 12 to 17 wherein the formulation is a tablet.

19. The formulation according to claim 18 which is suitable for oral administration.

20. A process for preparing the solid dispersion according to claim 8 comprising the steps of mixing ARN-509 and a poly(meth)acrylate copolymer in a suitable solvent and spray drying said mixture.

21. The process according to claim 20 wherein the suitable solvent is a mixture of dichloromethane and methanol.

22. The process according to claim 21 wherein the weight:weight ratio of dichloromethane to methanol in the mixture is 5:5.

23. The pharmaceutical formulation according to any one of claims 12 to 19 for use in the treatment of prostate cancer.

24. The pharmaceutical formulation for use according to claim 23 wherein the formulation is for oral administration.

25. A combination of a pharmaceutical formulation according to any one of claims 12 to 19 and another anticancer agent.

26. The combination according to claim 25 wherein the other anticancer agent is an androgen biosynthesis inhibitor.

27. The combination according to claim 25 wherein the other anticancer agent is abiraterone acetate.

28. The combination according to any one of claims 25 to 27 further comprising prednisone.

## Patentansprüche

1. Feste Dispersion, umfassend 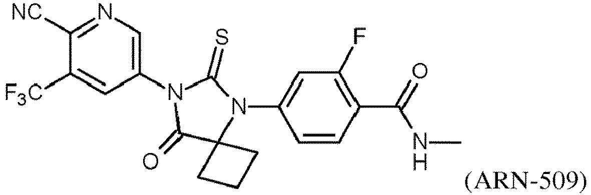 und ein Poly(meth)acrylatcopolymer.

2. Feste Dispersion nach Anspruch 1, wobei die Dispersion aus ARN-509 und einem Poly(meth)acrylatcopolymer besteht.

3. Feste Dispersion nach Anspruch 1 oder 2, wobei das Gewichtsverhältnis von ARN-509:Poly(meth)acrylatcopolymer in der festen Dispersion im Bereich von 1:1 bis 1:5 liegt.

4. Feste Dispersion nach Anspruch 3, wobei das Gewichtsverhältnis von ARN-509:Poly(meth)acrylatcopolymer in der festen Dispersion 1:2 beträgt.

5. Feste Dispersion nach einem der vorhergehenden Ansprüche, wobei ARN-509 in amorpher Form vorliegt.

6. Feste Dispersion nach einem der vorhergehenden Ansprüche, wobei es sich bei der Dispersion um eine feste Lösung handelt.

7. Feste Dispersion nach einem der vorhergehenden Ansprüche, wobei es sich bei dem Poly(meth)acrylatcopolymer um Poly(methacrylsäure-co-ethylacrylat) 1:1 handelt.

8. Feste Dispersion nach einem der vorhergehenden Ansprüche, erhältlich durch Sprühtrocknen.

9. Feste Dispersion nach einem der Ansprüche 1 bis 7, erhältlich durch Schmelzextrusion.

10. Partikel, bestehend aus einer wie in einem der vorhergehenden Ansprüche definierten festen Dispersion.

11. Partikel, umfassend eine wie in einem der Ansprüche 1 bis 9 definierte feste Dispersion.

12. Pharmazeutische Formulierung, umfassend einen pharmazeutisch unbedenklichen Träger und eine feste Dispersion nach einem der Ansprüche 1 bis 9.

13. Pharmazeutische Formulierung, umfassend einen pharmazeutisch unbedenklichen Träger und ein Partikel nach Anspruch 10 oder 11.

14. Formulierung nach Anspruch 12 oder 13, wobei die Formulierung 60 mg ARN-509 umfasst.

15. Formulierung nach Anspruch 12 oder 13, wobei die Formulierung 120 mg ARN-509 umfasst.

16. Formulierung nach Anspruch 12 oder 13, wobei die Formulierung 240 mg ARN-509 umfasst.

17. Formulierung nach einem der Ansprüche 12 bis 16, wobei das Gewicht der festen Dispersion im Bereich von 20 bis 40% des Gesamtgewichts der Formulierung liegt.

18. Formulierung nach einem der Ansprüche 12 bis 17, wobei es sich bei der Formulierung um eine Tablette handelt.

19. Formulierung nach Anspruch 18, welche sich für die orale Verabreichung eignet.

20. Verfahren zur Herstellung der festen Dispersion nach Anspruch 8, bei dem man ARN-509 und ein Poly(meth)acrylatcopolymer in einem geeigneten Lösungsmittel mischt und die Mischung sprühtrocknet.

21. Verfahren nach Anspruch 20, wobei es sich bei dem geeigneten Lösungsmittel um eine Mischung von Dichlormethan und Methanol handelt.

22. Verfahren nach Anspruch 21, wobei das Gewichtsverhältnis von Dichlormethan zu Methanol in der Mischung 5:5 beträgt.

23. Pharmazeutische Formulierung nach einem der Ansprüche 12 bis 19 zur Verwendung bei der Behandlung von Prostatakrebs.

24. Pharmazeutische Formulierung zur Verwendung nach Anspruch 23, wobei die Formulierung für die orale Verabreichung bestimmt ist.

25. Kombination einer pharmazeutischen Formulierung nach einem der Ansprüche 12 bis 19 und eines anderen Antikrebsmittels.

26. Kombination nach Anspruch 25, wobei es sich bei dem anderen Antikrebsmittel um einen Inhibitor der Androgenbiosynthese handelt.

27. Kombination nach Anspruch 25, wobei es sich bei dem anderen Antikrebsmittel um Abirateronacetat handelt.

28. Kombination nach einem der Ansprüche 25 bis 27, welche weiterhin Prednison umfasst.

## Revendications

1. Dispersion solide comprenant 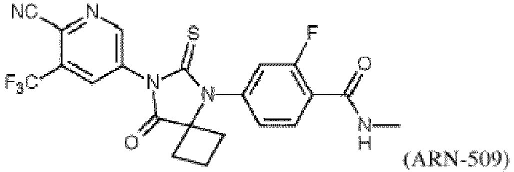 et un copolymère de poly(méth)acrylate.

2. Dispersion solide selon la revendication 1, la dispersion étant constituée d'ARN-509 et d'un copolymère de poly(méth)acrylate.

3. Dispersion solide selon la revendication 1 ou 2, le rapport poids-par-poids d'ARN-509:copolymère de poly(méth)acrylate dans la dispersion solide étant dans la plage de 1:1 à 1:5.

4. Dispersion solide selon la revendication 3, le rapport poids-par-poids d'ARN-509:copolymère de poly(méth)acrylate dans la dispersion solide étant de 1:2.

5. Dispersion solide selon l'une quelconque des revendications précédentes, l'ARN-509 étant présent sous forme amorphe.

6. Dispersion solide selon l'une quelconque des revendications précédentes, la dispersion étant une solution solide.

7. Dispersion solide selon l'une quelconque des revendications précédentes le copolymère de poly(méth)acrylate étant un poly(acide méthacrylique-co-acrylate d'éthyle) 1:1.

8. Dispersion solide selon l'une quelconque des revendications précédentes pouvant être obtenue par séchage par pulvérisation.

9. Dispersion solide selon l'une quelconque des revendications 1 à 7 pouvant être obtenue par extrusion à l'état fondu à chaud.

10. Particule constituée d'une dispersion solide telle que définie dans l'une quelconque des revendications précédentes.

11. Particule comprenant une dispersion solide telle que définie dans l'une quelconque des revendications 1 à 9.

12. Formulation pharmaceutique comprenant un support pharmaceutiquement acceptable et une dispersion solide selon l'une quelconque des revendications 1 à 9.

13. Formulation pharmaceutique comprenant un support pharmaceutiquement acceptable et une particule selon la revendication 10 ou 11.

14. Formulation selon la revendication 12 ou 13, la formulation comprenant 60 mg d'ARN-509.

15. Formulation selon la revendication 12 ou 13, la formulation comprenant 120 mg d'ARN-509.

16. Formulation selon la revendication 12 ou 13, la formulation comprenant 240 mg d'ARN-509.

17. Formulation selon l'une quelconque des revendications 12 à 16, le poids de la dispersion solide se situant dans la plage de 20 à 40 % du poids total de la formulation.

18. Formulation selon l'une quelconque des revendications 12 à 17, la formulation étant un comprimé.

19. Formulation selon la revendication 18 qui est appropriée pour une administration orale.

20. Procédé pour la préparation de la dispersion solide selon la revendication 8 comprenant les étapes de mélange d'ARN-509 et d'un copolymère de poly(méth)acrylate dans un solvant approprié et le séchage par pulvérisation dudit mélange.

21. Procédé selon la revendication 20, le solvant approprié étant un mélange de dichlorométhane et de méthanol.

22. Procédé selon la revendication 21, le rapport poids:poids de dichlorométhane à méthanol dans le mélange étant de 5:5.

23. Formulation pharmaceutique selon l'une quelconque des revendications 12 à 19 pour une utilisation dans le traitement du cancer de la prostate.

24. Formulation pharmaceutique pour une utilisation selon la revendication 23, la formulation étant destinée à une administration orale.

25. Combinaison d'une formulation pharmaceutique selon l'une quelconque des revendications 12 à 19 et d'un autre agent anticancéreux.

26. Combinaison selon la revendication 25, l'autre agent anticancéreux étant un inhibiteur de biosynthèse des androgènes.

27. Combinaison selon la revendication 25, l'autre agent anticancéreux étant l'acétate d'abiratérone.

28. Combinaison selon l'une quelconque des revendications 25 à 27 comprenant en outre de la prednisone.
